# EUROPÄISCHE PATENTANMELDUNG

(11) **EP 4 667 454 A1**
(43) Veröffentlichungstag der Anmeldung: **24.12.2025**
(21) Anmeldenummer: 24182893.8
(22) Anmeldetag: 18.06.2024
(51) Int. Cl.: C07C 267/00

(54) **VERFAHREN ZUR HERSTELLUNG VON TERTIÄREN ARALIPHATISCHEN CARBODIIMIDEN**

(71) Anmelder: LANXESS Deutschland GmbH, 50569 Köln (DE)
(72) Erfinder: LAUFER, Wilhelm, 67158 Ellerstadt (DE)

(57) **Zusammenfassung**

Die vorliegende Erfindung betrifft ein Verfahren zur Herstellung von tertiären araliphatischen Carbodiimiden aus araliphatischen Isocyanaten durch Umsetzung in Gegenwart von ein oder mehreren Diazaphospholidinoxiden, die dadurch erhältlichen Mischungen enthaltend araliphatische Carbodiimide und Diazaphospholidinoxide sowie deren Verwendung als Hydrolyseschutzmittel in PU und TPU.

## Beschreibung

Die vorliegende Erfindung betrifft das Gebiet von Carbodiimiden, deren Herstellung und Verwendung als Hydrolyseschutzmittel in Polyurethan(PU) und thermoplastischem Polyurethan (TPU).

Sterisch gehinderte Carbodiimide haben sich in vielen Anwendungen bewährt, z.B. als Hydrolyseschutzmittel für thermoplastische Kunststoffe, Ester-basierte Polyole, Polyurethane, Triglyceride und Schmierstofföle etc. bewährt. Insbesondere in Polyurethanen erzielen sterisch gehinderte Carbodiimide eine signifikant höhere Hydrolyseschutzwirkung verglichen mit sterisch weniger stark gehinderten Carbodiimiden.

Die Synthese der Carbodiimide erfolgt nach dem Stand der Technik ausgehend von Isocyanaten, die durch basische oder heterocyclische Katalyse unter COz-Abspaltung (poly)kondensiert (carbodiimidisiert) werden. Dabei können mono- oder polyfunktionelle Isocyanate zu monomeren oder polymeren Carbodiimiden umgesetzt werden.

Die üblicherweise verwendeten Katalysatoren sind Alkali- oder Erdalkaliverbindungen. Phosphorhaltige Katalysatoren für die Synthese von aliphatischen und aromatischen Carbodiimiden sind in FR1469946 beschrieben. Diese eignen sich jedoch nicht für die Herstellung von sterisch gehinderten Carbodiimiden, so dass sich hierfür Phospholenoxide speziell 1-Methyl-2-phospholen-1-oxid (MPO) durchgesetzt hat.

Die phosphorhaltigen Katalysatoren aus dem Stand der Technik haben den Nachteil, in den meisten PU-Anwendungen bereits in sehr geringen Konzentrationen (ppm) zu stören, da sie dort zu unerwünschten Nebenreaktionen führen.

Die vollständige Entfernung dieser phosphorhaltigen Katalysatoren ist technisch sehr kostspielig und erfolgt i. d. Regel durch aufwendige Destillationsstufen wie z. B. beschreiben in EP2897934.

Weitere basische und/oder heterogene Alternativkatalysatoren aus dem Stand der Technik wie im WO2015185645, WO 2016202781 oder WO 2022219110 beschrieben, zeigen meistens eine deutlich geringere Aktivität und sind ebenfalls im Produktionsmaßstab nur mit zusätzlichen kostspieligen Verfahrensstufen für das Auftrennen oder Zersetzen/ Entfernen der Restkatalysatoren nach der Reaktion für die Herstellung von Carbodiimiden zur Verwendung in PU-Anwendungen geeignet.

Aufgabe der vorliegenden Erfindung war es daher, ein verbessertes Verfahren bereitzustellen, welches die Herstellung von sterisch gehinderten Carbodiimiden in hohen Ausbeuten ermöglicht und einen die Polymerisation von PU nicht störenden Katalysator verwendet, so dass die erhaltenen sterische gehinderten Carbodiimide, ohne eine wesentliche Abtrennung des Katalysators bei der Herstellung von hydrolysestabilisierten Polyurethanen, insbesondere von TPU eingesetzt werden kann.

Überraschenderweise wurde nun gefunden, dass die vorgenannten Aufgaben gelöst wird durch ein Verfahren zur Herstellung von tertiären araliphatischen Carbodiimiden aus araliphatischen Isocyanaten, wobei die araliphatischen Isocyanate umgesetzt werden in Gegenwart von ein oder mehreren Diazaphospholidinoxiden der Formel (III), 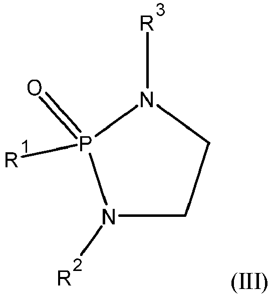 worin R¹, R², R³ unabhängig voneinander ausgewählt sind aus C₁ - C₆ -Alkyl, Cyclohexyl und Aryl und worin vorzugsweise R¹, R², R³ Methyl sind.

Der Begriff tertiäre araliphatische Carbodiimide bezeichnet dabei Verbindungen, bei denen eine oder beide, bevorzugt beide Seiten der Carbodiimidfunktion angebunden sind an je ein tertiäres Kohlenstoffatom, welches zwei Alkylgruppen und eine (ggf. substituierte) Arylgruppe trägt. Diese Carbodiimide weisen eine hohe sterische Abschirmung der Carbodiimidfunktion auf.

Die zur Herstellung der tertiären araliphatischen Carbodiimide zu verwendenden araliphatischen Isocyanate ergeben sich für den Fachmann aus der Struktur der Carbodiimide in naheliegender Weise.

Eine bevorzugte Ausführungsform der Erfindung ist ein Verfahren zur Herstellung von tertiären araliphatischen Carbodiimiden der Formel (la) 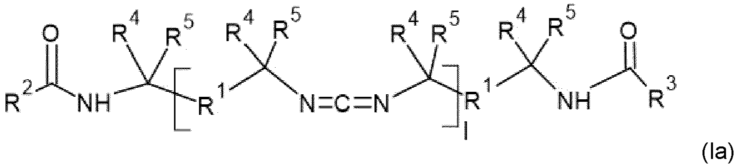 oder der Formel (Ib) 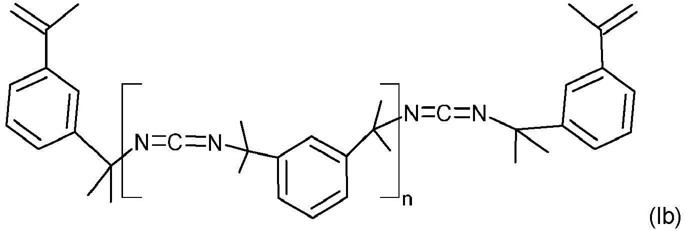 worin
- n: einer Zahl von 0 bis 20,bevorzugt 0 oder 3 bis 8, ganz besonders bevorzugt 0 oder 4 bis 5 entspricht,
- I: einer Zahl von 1 bis 20, bevorzugt von 3 bis 8, ganz besonders bevorzugt von 4 bis 5 entspricht,
- R¹: unabhängig voneinander ausgewählt sind unter Verbindungen der Formel laa, lab, lac oder lad, bevorzugt Verbindungen der Formel lab

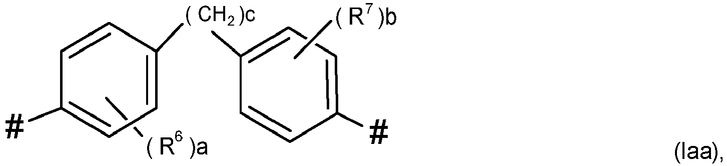

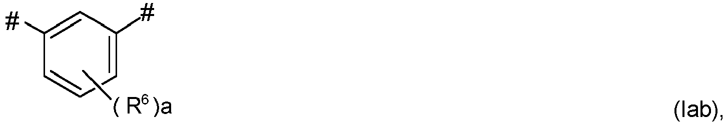

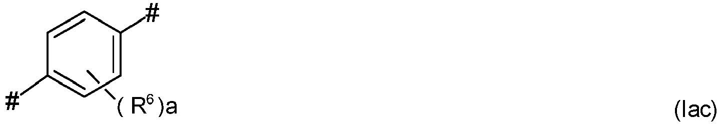

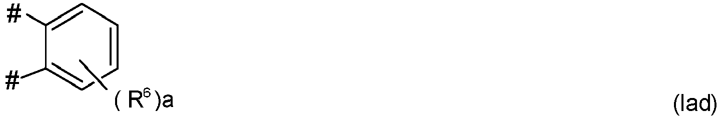

R⁴ und R⁵ unabhängig voneinander ausgewählt sind aus C₁ - C₄-Alkyl,
die Reste R⁶ und R⁷ unabhängig voneinander ausgewählt sind aus C₁ - C₄-Alkyl, C₁ - C₄-Alkoxy,
a, b, c für eine Zahl von 0 bis 4, bevorzugt 0 steht,
R² und R³ unabhängig voneinander ausgewählt sind aus -O-(C₁ - C₂₀-Alkyl), -O-(C₁ - C₂₀-Alkenyl), -O-(C₅ - C₂₀-Cycloalkyl), -O-Aryl, -O-(C₇ - C₂₀-Alkyaryl), -O-[(CH₂)ₖ-O]_{g} -R⁸, mit k= 1-3, g = 0-12 und R⁸= H oder C₁-C₄-Alkyl.

Eine weiter bevorzugte Ausführungsform der Erfindung ist ein Verfahren zur Herstellung von tertiären araliphatischen Carbodiimiden ausgewählt aus Bis[3-Isopropenyl- α,α-dimethylbenzyl]carbodiimid IIa 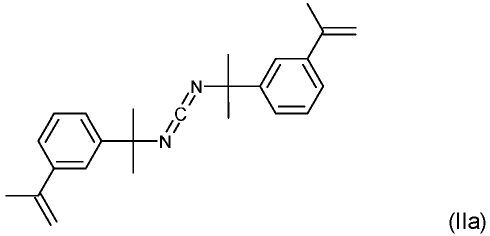 und Verbindungen der Formel IIb 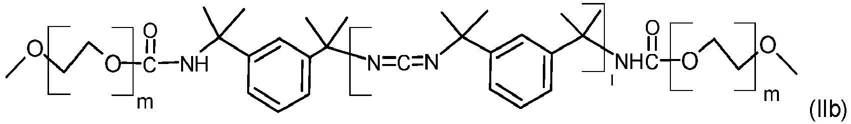 worin
- I: einer Zahl von 1 bis 20, bevorzugt von 3 bis 8, ganz besonders bevorzugt von 4 bis 5 entspricht und
- m: einer Zahl von 1 bis 20, bevorzugt von 3 bis 15, besonders bevorzugt von 5 bis 11 entspricht.

Eine weitere bevorzugte Ausführungsform der Erfindung betrifft ein Verfahren zur Herstellung von tertiären araliphatischen Carbodiimiden wobei als araliphatische Isocyanate 3-lsopro-penyphenyl-α,α-dimethylbenzylisocyanat (Illa) und/oder m-Tetramethylxylol-diisocyanat (IIIb) eingesetzt werden.

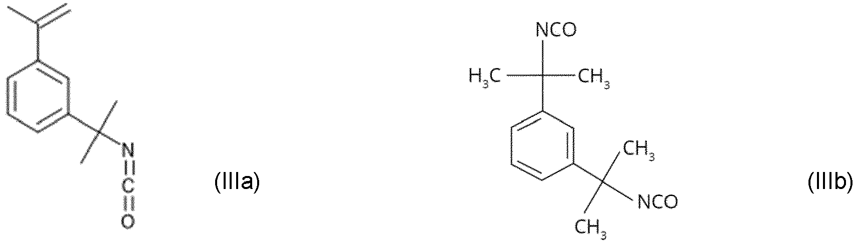

Beim erfindungsgemäßen Verfahren zur Herstellung von tertiären araliphatischen Carbodiimiden erfolgt die Umsetzung von Isocyanaten zu Carbodiimiden üblicherweise bei Temperaturen von 160 bis 220° C, vorzugsweise von 180 bis 200°C, besonders bevorzugt von 180 bis 190°C.

In einer bevorzugten Variante erfolgt die Umsetzung bei einem Druck von 10 - 1000 mbar bevorzugt 20 - 300 mbar besonders bevorzugt 50 - 100 mbar. Die Umsetzung kann in Anwesenheit oder Abwesenheit eines Stickstoffstromes durchgeführt werden.

Als Diazaphospholidinoxide der Formel III eignen sich Verbindungen, bei denen R¹, R², R³ unabhängig voneinander ausgewählt sind aus C₁ - C₆ -Alkyl, Cyclohexyl und Aryl, vorzugsweise C₁ - C₃ -Alkyl, Cyclohexyl und Phenyl und besonders bevorzugt 1,2,3-Trimethyl-2-oxo-1,3,2-diazaphospholidin)

Im einer bevorzugten Ausführungsform werden die Diazaphospholidinoxide in einer Konzentration von 0,5 bis 2 Gew.-%, bevorzugt von 0,8 bis 1,5 Gew.-% und besonders bevorzugt von 1 bis 1,2 Gew.-% jeweils bezogen auf die Gesamtmenge an araliphatischen Isocyanaten eingesetzt.

Die Carbodiimidisierung kann sowohl in Substanz als auch in einem Lösemittel durchgeführt werden. Als Lösemittel werden vorzugsweise Alkylbenzole, Paraffinöle, Polyethylenglykoldimethylether, Ketone oder Lactone eingesetzt.

In einer Ausführungsform der Erfindung zur Herstellung von endfuktionalisierten Carbodiimiden werden im Anschluss an die Carbodiimidisierung die endständigen freien Isocyanatgruppen der Carbodiimide mit aliphatischen und/oder aromatischen Alkoholen und/oder Alkoxypolyoxyalkylenalkoholen umgesetzt.

Zur Herstellung der erfindungsgemäßen Carbodiimiden der Formel Ia und Ilb wird die Carbodiimidisierung, wenn die Reaktionsmischung den gewünschten Gehalt an NCO-Gruppen, entsprechend einem mittleren Kondensationsgrad von I besitzt, üblicherweise gestoppt. In einer Ausführungsform der vorliegenden Erfindung wird hierzu die Temperatur der Reaktionsmischung auf 50 - 120 °C, bevorzugt 60 - 100 °C, besonders bevorzugt auf 80 - 90 °C reduziert und die freien endständigen Isocyanatgruppen der Carbodiimide endfunktionalisiert.

Bevorzugt werden die freien endständigen Isocyanatgruppen der Carbodiimide mit aliphatischen und/oder aromatischen Alkoholen und/oder Alkoxypolyoxyalkylenalkoholen vorzugsweise in einem geringem Überschuss an -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinndilaurat oder DOTL (Dioctylzinndilaurat), abreagiert. Das Stoffmengen-Verhältnis, Alkoholen und/oder Alkoxypolyoxyalkylenalkoholen zu Carbodiimiden liegt vorzugsweise bei 1,005 - 1,05 : 1, besonders bevorzugt bei 1,01 - 1,03 : 1, bezogen auf die vorhandenen N=C=O-Gruppen.

Zur Herstellung der erfindungsgemäßen Carbodiimide der Formen Ib mit n > 0 werden bevorzugt 3-Isopropenyphenyl-α,α-dimethylbenzylisocyanat (Illa) und m-Tetramethylxylol-diisocyanat (IIIb) in einem geeigneten Verhältnis vorgelegt und anschließend in Anwesenheit des Katalysators der Formel III carbodiimidisiert.

Ein weiterer Gegenstand der Erfindungen sind Mischungen enthaltend tertiäre araliphatische Carbodiimide und bezogen auf die araliphatischen Carbodiimide von 0,5 bis 2 Gew.-%, bevorzugt 0,8 bis 1,5 Gew.-%, besonders bevorzugt von 1 bis 1,2 Gew.-% Diazaphospholidinoxiden der Formel (III), welche durch die erfindungsgemäßen Verfahren zur Herstellung von tertiären araliphatischen Carbodiimiden in den oben beschriebenen Ausführungsvarianten erhältlich sind. Diese Mischungen können vorteilhaft verwendet werden zur Herstellung von hydrolysestabilisiertem Polyurethan (PU), bevorzugt von hydrolysestabilisiertem thermoplastischen Polyurethan (TPU). Diese Verwendung stellt somit einen weiteren Gegenstand der Erfindung dar.

Ein weiterer Gegenstand der vorliegenden Erfindung ist zudem ein Verfahren zu Herstellung von Polyurethanen (PU), bevorzugt thermoplastischen Polyurethanen dadurch gekennzeichnet, dass Polyole, bevorzugt Polyesterpolyole mit Polyisocyanaten gegebenenfalls in Anwesenheit von PU-Katalysatoren und Hilf- und oder Zusatzstoffen in Gegenwart des erfindungsgemäßen Carbodiimides zu Polyurethanen umgesetzt werden.

Die Herstellung der Polyurethane erfolgt dabei vorzugsweise, wie in WO 2005/111136 A1 beschrieben.

Polyurethane entstehen durch Polyadditionsreaktion von Polyisocyanaten mit mehrwertigen Alkoholen, den Polyolen, nahezu quantitativ. Die Verknüpfung erfolgt durch die Reaktion einer Isocyanatgruppe (-N=C=O) eines Moleküls mit einer Hydroxygruppe (-OH) eines anderen Moleküls unter Bildung einer Urethangruppe (-NH-CO-O-).

Der Reaktionsverlauf zwischen Diisocyanat und Polyol ist abhängig von dem Molverhältnis der Komponenten. Zwischenstufen mit gewünschtem Durchschnittsmolekulargewicht und gewünschten Endgruppen können durchaus erhalten werden. Diese Zwischenstufen können dann zu einem späteren Zeitpunkt mit einem Diol oder Diamin umgesetzt (kettenverlängert) werden, wobei dann das gewünschte Polyurethan bzw. Polyurethan-Polyharnstoff-Hybrid gebildet wird. Die Zwischenstufen werden im Allgemeinen als Prepolymer bezeichnet.

Geeignete Polyole für die Herstellung von Prepolymeren sind Polyalkylenglykolether, Polyetherester oder Polyester mit endständigen Hydroxylgruppen (Polyesterpolyole).

Bei den Polyolen im Sinne der Erfindung handelt es sich um Verbindungen, die vorzugsweise ein Molekulargewicht in (g/mol) von bis zu 2000, bevorzugt im Bereich von 500 bis 2000 und besonders bevorzugt im Bereich von 500 bis 1000 aufweisen.

Der Begriff Polyol im Sinne der Erfindung umfasst dabei sowohl Diole als auch Triole, wie auch Verbindungen mit mehr als drei Hydroxylgruppen je Molekül. Die Verwendung von Triolen ist besonders bevorzugt.

Bevorzugte Polyole sind Polyesterpolyole und/oder Polyetheresterpolyole.

Vorteilhaft ist es, wenn das Polyol eine OH-Zahl von bis zu 200, vorzugsweise zwischen 20 und 150 und besonders bevorzugt zwischen 50 und 115, aufweist.

Insbesondere eignen sich Polyesterpolyole, die Reaktionsprodukte von verschiedenen Polyolen mit aromatischen oder aliphatischen Dicarbonsäuren und/oder Polymeren von Lactonen sind.

Bevorzugt hierbei sind aromatische Dicarbonsäuren, welche zur Bildung geeigneter Polyesterpolyole verwendet werden können. Besonders bevorzugt sind hier Terephthalsäure, Isophthalsäure, Phthalsäure, Phtalsäureanhydrid sowie substituierte Dicarbonsäureverbindungen mit Benzolkern.

Als aliphatische Dicarbonsäuren sind solche bevorzugt, welche zur Bildung geeigneter Poylesterpolyole verwendet werden können, besonders bevorzugt Sebacinsäure, Adipinsäure und Glutarsäure.

Als Polymere von Lactonen sind solche bevorzugt, welche zur Bildung geeigneter Poylesterpolyole verwendet werden können, besonders bevorzugt Polycaprolacton.

Sowohl bei den Dicarbonsäuren als auch bei den Polymeren von Lactonen handelt es sich um handelsübliche Substanzen.

Besonders bevorzugt sind auch solche Polyole, welche zur Bildung geeigneter Polyesterpolyole verwendet werden können, ganz besonders bevorzugt Ethylenglykol, Butandiol, Neopentylglykol, Hexandiol, Propylenglykol, Dipropylenglykol, Diethylenglykol und Cyclohexandimethanol.

In einer weiteren bevorzugten Ausführungsform der Erfindung handelt es sich bei den Polyolen um Polyetheresterpolyole.

Hierfür sind die Reaktionsprodukte von verschiedenen vorhergehend genannten Polyolen mit aromatischen oder aliphatischen Dicarbonsäuren und/oder Polymeren von Lactonen (z.B. Polycaprolacton) bevorzugt.

Bei den im Sinne der Erfindungen eingesetzten Polyolen handelt es sich um handelsübliche Verbindungen, die bei der Firma Covestro Deutschland AG unter dem Handelsnamen Baycoll^{®} oder Desmophen^{®} erhältlich sind.

Als Diisocyanate sind aromatische und aliphatische Diisocyanate bevorzugt. Besonders bevorzugt sind Toluol-2,4-diisocyanat, Toluol-2,6-diisocyanat, Phenylendiisocyanat, 4,4-Diphenylmethandiisocyanat, Methylen-bis(4-phenylisocynat), Naphtalen-1,5-diisocyanat, Tetramethylen-1,4.diisocyanat und/oder Hexamethylen-1,6-diisocyanat, ganz besonders bevorzugt Toluol-2,4-diisocyanat und Toluol-2,6-diisocyanat.

Als Diisocyanate können handelsübliche Verbindungen eingesetzt werden, beispielsweise die bei der Firma Covestro Deutschland AG unter dem Handelsnamen Desmodur^{®} vertriebenen Verbindungen.

In einer weiteren Ausführungsform der Erfindung enthält die Zusammensetzung zusätzlich mindestens ein Diamin und/oder Diol.

Als Diamine, welche für die Kettenverlängerung eingesetzt werden, sind 2-Methylpropyl-3,5-diamino-4-chlorobenzoat, Bis-(4,4'-amino-3-chlorophenyl)-methan, 3,5-Dimethylthio-2,4-toluylendiamin, 3,5-Dimethylthio-2,4-toluylendiamin, 3,5-Diethyl-2,4-toluylendiamin, 3,5-Diethyl-2,6-toluylendiamin, 4,4'-Methylen-bis-(3-chloro-2,6-diethylanilin) und 1,3-Propandiol-bis(4-aminobenzoat) bevorzugt.

Als Diole sind Butandiol, Neopentylglykol, Hexandiol, Propylenglykol, Dipropylenglykol, Diethylenglykol und/oder Cyclohexandimethanol bevorzugt.

Als Diamine oder Diole können handelsübliche Verbindungen eingesetzt werden, beispielsweise die bei der Firma Lanxess Deutschland GmbH unter dem Handelsnamen Addolink^{®} vertriebenen Verbindungen.

Als Katalysatoren werden vorzugsweise Dibutylzinndilaurate oder Triethylendiamin in Dipropylenglycol eingesetzt.

Als PU-Katalysatoren können handelsübliche Verbindungen eingesetzt werden, beispielsweise die bei der Fa. Lanxess Deutschland GmbH unter dem Handelsnamen Addocat^{®} vertriebenen Verbindungen.

In einer bevorzugten Ausführungsform der vorliegenden Erfindung wird die erfindungsgemäße Mischung enthaltend tertiäres araliphatisches Carbodiimid und Diazaphospholidinoxid eingesetzt in einer Menge von 0,1 bis 2 Gew.-%, bevorzugt 0,5 bis 1,5 Gew.-%, bezogen auf die Gesamtmenge der Mischung zur Herstellung von Polyurethan.

Die dabei erhaltenen Polyurethane, insbesondere TPU zeichnen sich durch eine hervorragende Hydrolysebeständigkeit aus.

Gegenstand der vorliegenden Erfindung sind daher auch Zusammensetzungen, enthaltend
(a) mindestens ein tertiäres araliphatisches Carbodiimid, vorzugsweise ein Carbodiimid der Formel la oder Ib besonders bevorzugt der Formel Ila oder IIb,
(b) mindestens ein Diazaphospholidenoxide der Formel (III) und
(c) Mindestens ein Polyurethan, vorzugsweise thermoplastisches Polyurethan

In einer bevorzugten Ausführungsform enthält die Polyurethanzusammensetzung einen Anteil an Komponente (a) von 0,1 bis 2 Gew.-%, bevorzugt von 0,5 bis 1,5 Gew.-% und einen Anteil der Kompontente (b) von 0,0005 Gew.-% bis 0,04 Gew.-%, bevorzugt von 0,004 Gew.-% bis 0,02 Gew.-% jeweils bezogen auf die Gesamtzusammensetzung.

Gegenstand der vorliegenden Erfindung ist weiterhin ein Verfahren zur Herstellung von hydrolysestabilisiertem Polyurethan, bevorzugt von hydrolysestabilisiertem TPU, dadurch gekennzeichnet, dass man in einem ersten Schritt tertiäre araliphatische Carbodiimide durch ein erfindungsgemäßes Verfahren herstellt und diese ohne wesentliche Abtrennung von Diazaphospholidinoxiden der Formel (III) zu einem Reaktionsgemisch zur Herstellung eines Polyurethans oder TPU zugibt und anschließend das dadurch erhaltene Gemisch zu einem Polyurethan oder TPU umsetzt.

Der Ausdruck ohne wesentliche Abtrennung umfasst dabei solche Verfahrensschritte, die auf die Entfernung des Katalysators gerichtet sind, Nicht darunter fallen Reinigungsschritte insbesondere zur Abtrennung von Lösungsmitteln, welche die Menge des Katalysators in der im ersten Schritt erhaltenen Reaktionsmischung um weniger als 30 Gew.-%, vorzugsweise weniger als 50 Gew.-%, besonders bevorzugt weniger als 70 Gew.-% und meist bevorzugt um weniger als 90 Gew.-% verringern. In einer meist bevorzugten Ausführungsform der Erfindung wird das im ersten Schritt erhaltene, tertiäre araliphatische Carbodiimide und Diazaphospholidinoxiden der Formel (III) enthaltende Reaktionsgemisch ohne weitere Aufarbeitung eingesetzt.

Das Reaktionsgemisch zur Herstellung eines Polyurethans oder TPU enthält typischerweise Polyole, bevorzugt Polyesterpolyole, Polyisocyanate und ggf. einen oder mehrere Polyurethankatalysatoren.

Prinzipiell kann die Zugabe der im ersten Schritt erhaltenen, tertiäre araliphatische Carbodiimide und Diazaphospholidinoxiden der Formel (III) enthaltenden Mischung zum Reaktionsgemisch zur Herstellung eines Polyurethans oder TPU prinzipiell in jedem Stadium des Herstellungsverfahrens eines thermoplastischen Polyurethans erfolgen, also auch in einem Stadium, in dem die Polymerisation schon begonnen hat oder wenn Prepolymere verwendet werden.

Ein weiterer Gegenstand der vorliegenden Erfindung ist die Verwendung von tertiäre araliphatische Carbodiimide und Diazaphospholidinoxide der Formel (III) enthaltenden Mischungen in Verfahren zur Herstellung von hydrolysestabilisierten Polyurethanen und TPU.

Ein weiterer Gegenstand der vorliegenden Erfindung ist zudem die Verwendung der tertiäre araliphatische Carbodiimide und Diazaphospholidinoxide der Formel (III) enthaltenden Mischung in thermoplastischen Polyurethanen (TPU), in Polyurethan-Elastomeren, in PU-Klebstoffen, in PU-Gießharzen, in PU-Schäumen oder in PU-Beschichtungen für Holz, Leder, Kunstleder und Textilien als Schutz gegen hydrolytischen Abbau.

Die nachfolgenden Beispiele dienen der Erläuterung der Erfindung, ohne dabei limitierend zu wirken.

### Ausführungsbeispiele:

| | |
|---|---|
| Beispiel 1: | Herstellung eines sterisch gehinderten aromatischen Carbodiimides durch die Umsetzung der Verbindung 1,3,5-Triisopropyl-2,4-phenyldiisocyanat (TRIDI) mit Methylphospholenoxid (MPO), (Vergleich) |
| Beispiel 2: | Herstellung eines sterisch gehinderten araliphatischen Carbodiimides durch die Umsetzung der Verbindung m-Tetramethylxyloldiisocyanat (TMXDI) mit Methylphospholenoxid (MPO), (Vergleich) |
| Beispiel 3: | Herstellung eines sterisch gehinderten aromatischen Carbodiimides durch die Umsetzung der Verbindung 1,3,5-Triisopropyl-2,4-phenyldiisocyanat (TRIDI) mit 1,2,3-Trimethyl-2-oxo-1,3,2-diazaphospholidin, (Vergleich) |
| **Beispiel 4:** | Herstellung eines aliphatischen Carbodiimides durch die Umsetzung der Verbindung Dicyclohexylmethanl-4,4'-diisocyanat (H₁₂MDI) mit 1,2,3-Trimethyl-2-oxo-1,3,2-diazaphospholidin, (Vergleich) |
| Beispiel 5: | Herstellung eines sterisch gehinderten araliphatischen Carbodiimides durch die Umsetzung der Verbindung m-Tetramethylxyloldiisocyanat (TMXDI) mit 1,2,3-Trimethyl-2-oxo-1,3,2-diazaphospholidin , (erfindungsgemäß). |

Allgemeine Herstellvorschrift für die Beispiele 1 - 5:
Es wurden 30 g der Isocyanat-Gruppen-enthaltenden Verbindung m-Tetramethylxylol-diisocyanat (TMXDI), Dicyclohexylmethanl-4,4'-diisocyanat (H₁₂MDI) bzw. 1,3,5-Triisopropyl-2,4-phenyldiisocyanat (TRIDI) in einen 100 ml-Dreihalskolben eingewogen, der mit Innenthermometer, Rückflusskühler und Schutzgaseinlass ausgestattet war, und daraufhin wurde die Gew.% des jeweiligen Katalysators gemäß Tabelle 1 zugefügt. In der Aufheizphase gab man einen leichten Argonstrom über die Dampfphase. Bei beginnender CO₂-Entwicklung wurde das Schutzgas abgestellt. Man ließ bei 180 °C unter Rühren carbodiimidisieren bis der Ziel-Gehalt von NCO von 12 Gew.% erreicht wurde. Die Bildung von Carbodiimid-Gruppen (NCN) wurde mittels IR-Spektrum bestätigt. Danach wurde die Reaktionsmischung abgekühlt und die restlichen NCO-Gruppen mit Methylpolyethylenglykol MPEG (Mw ca. 500 g/mol) abreagiert.

**Tabelle 1: Reaktionszeiten für die Synthese des Carbodiimides**

| **Bsp.** | **Katalysator** | **T [°C]** | **Isocyanat** | **Menge Katalysator [Gew.%]** | **Dauer [h]** | **IsocyanatGehalt [Gew.%]** |
|---|---|---|---|---|---|---|
| **1** | Methylphospholenoxid (MPO) - Vergleich | 180 | TRIDI | 0,02 | 7 | ca. 12 |
| **2** | Methylphospholenoxid (MPO) - Vergleich | 180 | TMXDI | 0,2 | 20 | ca. 12 |
| **3** | 1,2,3-Trimethyl-2-oxo-1,3,2-diazaphospholidin - Vergleich | 180 | TRIDI | 1,0 | 35 | > 17 (keine weitere Reaktion) |
| **4** | 1,2,3-Trimethyl-2-oxo-1,3,2-diazaphospholidin - Vergleich | 180 | H₁₂MDI | 1,0 | > 50 | ca. 15 |
| **5** | 1,2,3-Trimethyl-2-oxo-1,3,2-diazaphospholidin - erfindungsgemäß | 180 | TMXDI | 1,0 | 20 | ca. 12 |

Überraschenderweise zeigt das verwendete Diazaphospholidinoxid für die Carbodiimidisierung von TMXDI eine hohe Katalysatoraktivität und führt nach 12 Stunden Reaktionszeit zu ähnlichen NCO-Gehalte, wohingegen die Katalysatoraktivität für das sterisch gehinderte aromatische Isocyanat TRIDI und das sterisch gehinderte aliphatische Isocyanat H₁₂MDI nieder war.

Zudem muss der erfindungsgemäße Katalysator im Vergleich zum Stand der Technik nicht durch Filtration, Extraktion, thermische Zersetzung und/oder Destillation nach der Synthese abgetrennt werden und kann direkt auch in PU-Anwendungen wie z. B. zur Stabilisierung von thermoplastischen Polyurethanelastomeren oder Polyurethanschäumen eingesetzt werden.

### PU-Reaktivitätstest auf störende Nebeneffekte

1 Teil Polycarbodiimid und 9 Teile 4,4'-Methylen-di(phenylisocyanat) (MDI) wurden in einen 250 ml Vierhalskolben vorlegt. Anschließend wurde mit Stickstoff inertisiert. Anschließend wurde die Mischung auf 80 °C erwärmt und gerührt. Es wurde geprüft, ob die Mischung schäumt bzw. ob Gasentwicklung erfolgt. Nach 24 Stunden wurde zusätzlich an der Endprobe beurteilt, ob die Viskosität angestiegen war.

Die Eigenschaften der in Beispiel 6 und 7 erhaltenen polymeren Carbodiimide sind in Tabelle 2 wiedergegeben:

**Tabelle 2: PU-Reaktivitätstest**

| Beispiel | Carbodiimid | PU-Reaktivitätstest |
|---|---|---|
| 6 | CDI aus Beispiel 2 (Vgl.) | Starke Gasentwicklung, Viskositätsanstieg |
| 7 | CDI aus Beispiel 5 (Erf.) | Keine Gasentwicklung, kein Viskositätsanstieg |

| | | |
|---|---|---|
| Vgl. = Vergleichsbeispiel; Erf. = erfindungsgemäß | | |

### Versuche in PU-Schmelzklebstoff

Der Schmelzklebstoff wurde wie folgt hergestellt:
Dynacoll^{®}, welcher kommerziell erhältlich ist von der Firma Evonik, ist ein linearer Copolyester mit primären Hydroxylfunktionen und einem mittleren molekularen Gewicht.

Zunächst wird der Copolyester für 30 Minuten und bei 120 °C evakuiert. Anschließend erfolgt die Zugabe von 11,67 Gew.% Diphenylmethandiisocyanat (MDI), bezogen auf die Gesamtformulierung und es wird für 60 Minuten bei 120 °C umgesetzt. Anschließend werden die jeweiligen, in der Tabelle 3 angegebenen Carbodiimide in den Schmelzklebstoff eingearbeitet und eine Einwirkzeit der Additive von 1 Stunde sichergestellt. Die so hergestellten und additivierten Schmelzklebstoffe (Hot-Melt) wurden in einer Kartusche einer Temperaturalterung bei 130 °C für 48 Stunden unterworfen. Die Temperaturalterung wurde in einer Alukartusche (Licht- und Feuchtigkeitsdicht) abgefüllt und im Umluftofen für 48 Stunden bei 130 °C gealtert.

Nach der Alterung wurde das Schäumverhalten der Proben visuell beurteilt.

Die Ergebnisse der Messungen sind in der Tabelle 3 zusammengestellt:

**Tabelle 3:**

| **Beispiel** | **Carbodiimid** | **Schäumverhalten** |
|---|---|---|
| **8** (Erf.) | aus Beispiel 5 | Kein Schaum, Keine oder sehr geringe Blasenbildung |
| **9** (Vgl.) | aus Beispiel 2 | Schaum- bzw. starke Blasenbildung |

| | | |
|---|---|---|
| V = Vergleichsbeispiel; Erf. = erfindungsgemäß | | |

## Patentansprüche

1. Verfahren zur Herstellung von tertiären araliphatischen Carbodiimiden aus araliphatischen Isocyanaten, wobei die araliphatischen Isocyanate umgesetzt werden in Gegenwart von ein oder mehreren Diazaphospholidinoxiden der Formel (III), 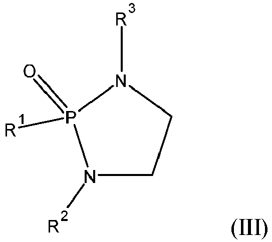 worin R¹, R², R³ unabhängig voneinander ausgewählt sind aus C₁ - C₆ -Alkyl, Cyclohexyl und Aryl und worin vorzugsweise R¹, R², R³ Methyl sind.

2. Verfahren zur Herstellung von tertiären araliphatischen Carbodiimiden gemäß Anspruch 1, wobei die tertiären araliphatischen Carbodiimide der Formel (la) 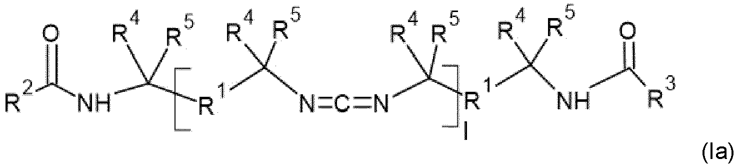 oder der Formel (Ib) 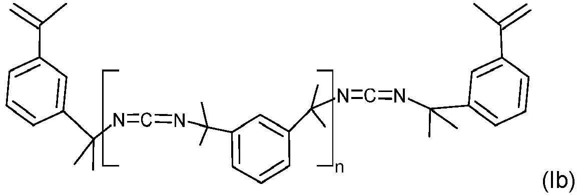 entsprechen, worin
n einer Zahl von 0 bis 20,bevorzugt 0 oder 3 bis 8, besonders bevorzugt 0 oder 4 bis 5 entspricht,
I einer Zahl von 1 bis 20, bevorzugt von 3 bis 8, besonders bevorzugt von 4 bis 5 entspricht,
R¹ unabhängig voneinander ausgewählt sind unter Verbindungen der Formel laa, lab, lac oder lad, bevorzugt Verbindungen der Formel lab 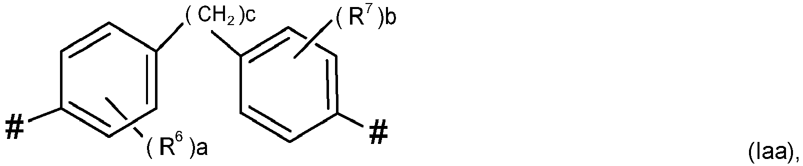 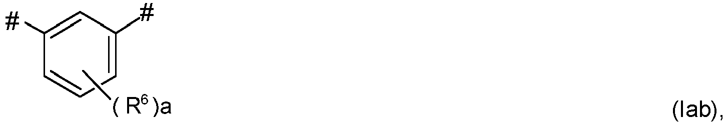 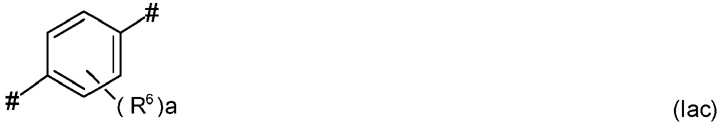 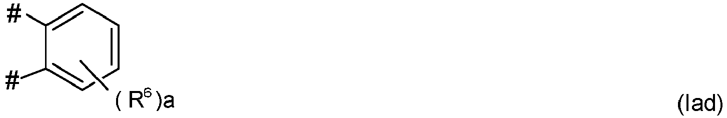
R⁴ und R⁵ unabhängig voneinander ausgewählt sind aus C₁ - C₄-Alkyl,
die Reste R⁶ und R⁷ unabhängig voneinander ausgewählt sind aus C₁ - C₄-Alkyl, C₁ - C₄-Alkoxy,
a, b, c für eine Zahl von 0 bis 4, bevorzugt 0 steht,
R² und R³ unabhängig voneinander ausgewählt sind aus -O-(C, - C₂₀-Alkyl), -O-(C, - C₂₀-Alkenyl), -O-(C₅ - C₂₀-Cycloalkyl), -O-Aryl, -O-(C₇ - C₂₀-Alkyaryl), -O-[(CH₂)ₖ-O]_{g} -R⁸, mit k= 1-3, g = 0-12 und R⁸= H oder C₁-C₄-Alkyl.

3. Verfahren zur Herstellung von tertiären araliphatischen Carbodiimiden gemäß einem oder mehreren der Ansprüche 1 bis 3, wobei die Carbodiimide ausgewählt sind aus Bis[3-Isopropenyl- α,α-dimethylbenzyl]carbodiimid (IIa) 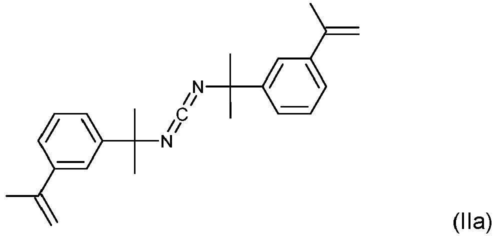 und Verbindungen der Formel Ilb 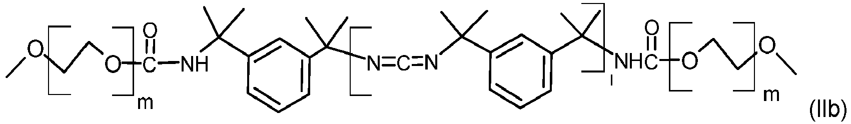 worin
I einer Zahl von 1 bis 20, bevorzugt von 3 bis 8, ganz besonders bevorzugt von 4 bis 5 entspricht und
m einer Zahl von 1 bis 20, bevorzugt von 3 bis 15, besonders bevorzugt von 5 bis 11 entspricht.

4. Verfahren zur Herstellung von tertiären araliphatischen Carbodiimiden gemäß einem oder mehreren der Ansprüche 1 bis 3, wobei als araliphatische Isocyanate 3-lsopropenyphenyl-α,α-dimethylbenzylisocyanat und/oder m-Tetramethylxyloldiisocyanat eingesetzt werden.

5. Verfahren zur Herstellung von tertiären araliphatischen Carbodiimiden gemäß einem oder mehreren der Ansprüche 1 bis 4, wobei die Umsetzung bei Temperaturen von 160 bis 220° C, vorzugsweise von 180 bis 200°C, besonders bevorzugt von 180 bis 190°C erfolgt.

6. Verfahren zur Herstellung von tertiären araliphatischen Carbodiimiden gemäß einem oder mehreren der Ansprüche 1 bis 5, wobei bei die Umsetzung in Anwesenheit von von 0,5 bis 2 Gew.-%, bevorzugt 0,8 bis 1,5 Gew.-%, besonders bevorzugt von 1 bis 1,2 Gew.-% bezogen auf die Gesamtmenge an araliphatischen Isocyanaten erfolgt.

7. Verfahren zur Herstellung von tertiären araliphatischen Carbodiimiden gemäß einem oder mehreren der Ansprüche 1 bis 6, wobei nach Carbodiimidisierung der araliphatischen Isocyanate zu Carbodiimiden die endständigen freien Isocyanatgruppen der Carbodiimide mit aliphatischen und/oder aromatischen Alkoholen und/oder Alkoxypolyoxyalkylenalkoholen reagiert werden.

8. Mischungen enthaltend tertiäre araliphatische Carbodiimide und bezogen auf die araliphatischen Carbodiimide von 0,5 bis 2 Gew.-%, bevorzugt 0,8 bis 1,5 Gew.-%, besonders bevorzugt von 1 bis 1,2 Gew.-% Diazaphospholidinoxiden der Formel (III), 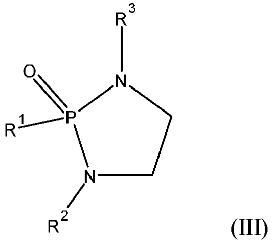
worin R¹, R², R³ unabhängig voneinander ausgewählt sind aus C₁ - C₆ -Alkyl, Cyclohexyl und Aryl und vorzusgweise R¹, R², R³ Methyl sind,
erhältlich gemäß einem oder mehrerer der Ansprüche 1 bis 7.

9. Verwendung von Mischungen gemäß Anspruch 8 zur Herstellung von hydrolysestabilisiertem Polyurethan (PU), bevorzugt hydrolysestabilisiertem thermoplastischem Polyurethan (TPU).

10. Verfahren zur Herstellung von hydrolysestabilisiertem Polyurethan (PU), bevorzugt von hydrolysestabilisiertem thermoplastischen Polyurethan (TPU), **dadurch gekennzeichnet, dass** man in einem ersten Schritt tertiäre araliphatische Carbodiimide gemäß einem oder mehreren der Ansprüche 1 bis 7 herstellt und diese ohne wesentliche Abtrennung des oder der Diazaphospholidinoxide der Formel (III) in einem weiteren Schritt mit Polyolen, bevorzugt Polyesterpolyolen, Polyisocyanaten und ggf. einem oder mehreren Polyurethankatalysatoren in Kontakt gebracht und zu hydrolysestabilisiertem Polyurethan (PU), bzw. hydrolysestabilisiertem thermoplastischen Polyurethan (TPU) umgesetzt wird.

11. Polyurethan erhältlich durch das Verfahren gemäß Anspruch 10.

12. Thermoplastisches Polyurethan erhältlich durch das Verfahren gemäß Anspruch 11

13. Zusammensetzungen enthaltend
(a) mindestens ein tertiäres araliphatisches Carbodiimid, vorzugsweise ein Carbodiimid der Formel Ia oder Ib, besonders bevorzugt der Formel Ila oder IIb,
(b) mindestens ein Diazaphospholidenoxide der Formel (III) und
(c) mindestens ein Polyurethan, vorzugsweise thermoplastisches Polyurethan.

14. Zusammensetzung gemäß Anspruch 13, worin der Anteil an Komponente (a) von 0,1 bis 2 Gew.-%, bevorzugt von 0,5 bis 1,5 Gew.-% und einen Anteil der Komponente (b) von 0,0005 Gew.-% bis 0,04 Gew.-%, bevorzugt von 0,004 Gew.-% bis 0,02 Gew.-% beträgt, jeweils bezogen auf die Gesamtzusammensetzung.
